## Europäisches Patentamt

(19) **European Patent Office**

**Office européen des brevets**

(11) Veröffentlichungsnummer: **0 238 839**
**A2**

# (12) EUROPÄISCHE PATENTANMELDUNG

(21) Anmeldenummer: **87102219.0**

(22) Anmeldetag: **17.02.87**

(51) Int. Cl.4: **A61L 15/03**

(30) Priorität: **27.02.86 DE 3606265**

(43) Veröffentlichungstag der Anmeldung:
**30.09.87 Patentblatt 87/40**

(84) Benannte Vertragsstaaten:
**AT BE CH DE ES FR GB IT LI LU NL SE**

(71) Anmelder: **Röhm Pharma GmbH**
**Dr.-Otto-Röhm-Strasse 2-4**
**D-6108 Weiterstadt 1(DE)**

(72) Erfinder: **Petereit, Hans-Ulrich**
**Lortzingstrasse 22**
**D-6100 Darmstadt(DE)**
Erfinder: **Zeiss, Gerhard, Dr.**
**Sudetenstrasse 12 c**
**D-6080 Gross-Gerau(DE)**

(54) **Wundauflage auf Polysaccharidbasis als Träger therapeutisch wirksamer, nicht-immobilisierter Enzyme und mit hoher Saugfähigkeit.**

(57) Die Erfindung betrifft eine im Normalfall als Flächengebilde ausgestaltete Wundauflage auf Polysaccharidbasis, insbesondere Cellulose, die infolge ihrer schwammartigen Struktur eine hohe Saugfähigkeit besitzt und nicht-immobilisierte Enzyme für die Wundtherapie enthält.

EP 0 238 839 A2

## Gebiet der Erfindung

Die Erfindung betrifft Flächengebilde auf Polysaccharidbasis, wie z.B. der Cellulose, als Träger therapeutisch wirksamer nicht-immobilisierter Enzyme, die zur enzymatischen Wundreinigung als Therapiemaßnahme eingesetzt werden.

## Stand der Technik

Für die Wundreinigung, die eine Voraussetzung für das schnelle Abheilen von Wunden ist, und die in einem Entfernen von nekrotischem Gewebe und Eiter besteht, hat sich neben chirurgischem Tun und der medikamentösen Behandlung mit Antiseptika oder Antibiotika, die enzymatische Wundreinigung bewährt.

In der topischen Enzymtherapie von Wunden, die durch Verletzungen, Verbrennungen, Hautkrankheiten u.a. verursacht sind, werden zur direkten Abdeckung der betroffenen Körperpartien Verbandmaterialien verwendet, die lockere bis feste Fasermassen, wie z.B. Gewebe von faserbildenden Stoffen natürlicher und/oder synthetischer Art sind. An ihre Reinheit, Saugfähigkeit und Beständigkeit bei der Einwirkung von Sekreten und Medikamenten werden daher hohe Anforderungen gestellt.

Nach den Ausführungen in Deutsche Medizinische Wochenschrift, 85 (1960), 672, werden durch lokale Anwendungen von Enzymen, wie z.B. mit dem Enzympräparat Jatrosin ®, auf Kompressen oder Mull-Lagen und Schaffung einer feuchten Kammer durch Abdeckung mit einer Plastikfolie, bei tiefgehenden Gewebsnekrosen raschere Heilungen im Vergleich zu anderen bekannten nekrolytischen Wundapplikationen erreicht.

Für den Abbau nekrotischen Gewebes eignen sich vor allem proteolytische Enzyme wie Trypsin, Chymotrypsin, Papain, Ficin, Bromelain sowie das aus Bakterien gewonnene Subtilisin. Zum Auflösen von Eiter werden vor allem Ribonucleasen und Desoxyribonucleasen, wie die Streptodornase, verwendet. Streptodornase eignet sich in Kombination mit Streptokinase auch zur Reinigung von Wunden von nekrotischem Gewebe und Fibrin. Mit Hilfe des Enzyms Hyaluronidase kann das Eindringen lokal applizierter Wirkstoffe in das Gewebe erleichtert werden.

Der beschleunigte Heilungsvorgang durch Therapie mit solchen äußerlich angewendeten Enzymen beruht auf dem dadurch schnell in Gang kommenden Abbau von Nekroseanteilen und dem dadurch verstärkten Selbstheilungsprozeß. Es kommt in der Folge zur raschen Ausfüllung der Gewebedefekte mit Granulationsgewebe und damit auch zu einer frühen Epithelisierung.

Die praktische Durchführung der Enzym-Therapie besteht in der Abdeckung der Wunde aus einem mit Enzymen präparierten Zuschnitt aus ca. 4 bis 6 Lagen Verbandmull, wobei im Interesse einer vollen Präparatwirkung die Schaffung einer feuchten Kammer, die an den Rändern noch mit Zinkpaste abgedichtet sein kann, erforderlich ist.

Nach der DE-A 31 39 089 werden als Enzymträger Flächengebilde auf Proteinbasis vorgeschlagen, die in Anlehnung an den Stand der Technik beim Vorgehen mit Flächengebilden auf Polysaccharidbasis angewendet werden können. Besonders günstig soll danach die Anwendung von Flächengebilden auf Kollagenbasis sein, da Kollagen ein hohes Wasseraufnahme-und Bindungsvermögen hat.

Trotz dieser günstigeren heilungsfördernden Voraussetzungen, hat sich diese Applikationsform wegen des sehr hohen Preises von Kollagengeweben nicht durchgesetzt. Die Preisrelation zwischen Kollagengeweben und herkömmlichen, in der Praxis angewendeten Geweben liegt bei etwa 1 000 : 1.

## Aufgabe und Lösung

Es bestand daher die Aufgabe eine Applikationsform für die topische Anwendung von Enzymen in der Therapie zu finden, die möglichst wenig in den Heilungsprozeß des Organismus eingreift, diesen jedoch noch mehr begünstigt, als dies bisher nach dem Stand der Technik in breitem Umfang durchgeführt werden konnte.

Es wurde gefunden, daß diese Aufgabe relativ weitgehend dadurch zu lösen ist, durch die kombinierte Wirkung von bekannten nekrolysierenden Enzymen und einer schwammartigen Cellulosezubereitung als Enzymträger und zur Abdeckung der Wunde. Diese sorgt durch ein hohes Adsorptionsvermögen für den raschen und ständigen Abtransport der verflüssigten Wundbestandteile.

Die Erfindung ist kostengünstig in einer breiten Anwendung durchführbar. Sie bringt vor allem eine weitere deutliche Beschleunigung der Wundreinigung und damit schnellere und bessere Wundausheilung und verringert praktische Schwierigkeiten, wie z.B. das Problem des kontinuierlichen Hinbringens der Enzyme an die Wunde, was jetzt durch die schwammartige Struktur der Abdeckung und der darin möglichen Lösungs-und Diffusionsvorgänge geregelt wird, oder den häufigen Verbandwechsel. Dies alles bringt merkliche Vereinfachungen in der Anwendung.

Durchführung der Erfindung

Die für die enzymatische und/oder chemische Wundreinigung neuartigen Verbandmaterialien sind textile Flächengebilde, die unter dem Begriff Vliesstoffe bekannt sind und eine weite praktische Anwendung gefunden haben. Nach verschiedenen Herstellungsverfahren für Vliesstoffe kann die Verwendung aller marktgängigen Fasern, sowohl von Naturfasern, wie Pflanzenfasern, oder auch von Chemiefasern dafür in Betracht kommen. Für die neuartige Enzym-Verbandmaterial-Kombination kommen vor allem solche Flächengebilde in Frage, die ganz oder im wesentlichen aus den preiswerten faserbildenden Rohstoffen auf Polysaccharidbasis, wie Cellulose oder Zellwolle, aufgebaut sind. Je nach Vorfertigungsgrad schwankt der Porositätsgrad dieser erfindungsgemäß anzuwendenden Flächengebilde zwischen 70 und 99, insbesondere 88 und 98 % (zum Begriff Porositätsgrad siehe Römpps Chemie-Lexikon, 7. Auflage, Seite 2784).

Im Vergleich zu den bisher üblich verwendeten Wundabdeckungen auf Collagenbasis liegt die Saugfähigkeit der erfindungsgemäßen Wundabdeckungen bei der Enzymtherapie um den Faktor 1,5 bis 5, insbesondere 1,5 bis 3,0 höher.

Die Saugfähigkeit der erfindungsgemäß zu verwendenden textilen Flächengebilde liegt, bezogen auf die Trockenmasse der Cellulose, bei 1500 bis 3000 Gew.-% Wasser. Sie wird beispielsweise bestimmt durch 5 minütiges Tränken eines 2 Stunden bei 100 Grad Celsius getrockneten und gewichtsmäßig bestimmten Flächenstücks in Wasser bei 20 Grad Celsius, anschließendem Abtupfen von nicht adsorbiertem Oberflächenwasser und folgender Wägung. Es ist dann:

$$\text{Saugfähigkeit (in Gew.-\% Wasser)} = \frac{\text{Gewicht des Gewebes mit Wasser getränkt}}{\text{Gewicht des Gewebes nach 100 Grad Celsius-Trocknung}} \times 100$$

Die infrage stehenden Enzyme können dem Polysaccharidträger vor oder bei der Applikation zugesetzt werden. Als Enzyme kommen die vorstehend genannten, insbesondere Proteasen in Betracht, aber auch die einschlägig verwendeten RN-asen und DN-asen und die Hyaluronidase. Die proteolytischen Enzyme werden mit Vorteil als Enzymkombinationen eingesetzt, wozu insbesondere der pankreatische Enzymkomplex zählt, der bekanntlich aus Trypsin, Chymotrypsin, verschiedenen Peptidasen und gegebenenfalls Begleitenzymen vom Typ der Amylase und Lipase besteht. Oder es werden Einzelenzyme, insbesondere Trypsin und Chymotrypsin, ferner Proteasen pflanzlichen Ursprungs, wie das Papain, das Bromelain und das Ficin, ferner die Proteasen mikrobiologischen Ursprungs, wie die Bakterienproteasen, z.B. das Subtilisin, in der neuartigen Kombination verwendet. (Vgl. Ullmanns Encyklopädie der techn. Chemie, 4. Auflage, Bd. 10, S. 555, Verlag Chemie, 1975).

Das Einbringen der Enzyme auf bzw. in die porösen textilartigen Wundauflagen geschieht z.B. aus standardisierten Enzymlösungen, z.B. in physiologischer Kochsalzlösung, nach bekannten Verfahren, die den hygienischen und antiseptischen Anforderungen genügen. Auch bei der Lagerung und Handhabung der Wundauflagen mit oder ohne Enzymauftrag ist auf Reinheit und Sterilität peinlich zu achten. Diese Bedingungen sind von dem Fachmann mit geläufigen Mitteln zu erreichen.

Außer Enzymen können die erfindungsgemäßen Wundauflagen noch Wirkstoffe, wie Arzneimittel, Antibiotika, Hormone, Salbenbestandteile u.ä. enthalten.

Das zur Abdeckung zu verwendende Flächengebilde kann in Schichtdicken von 0,1 bis 10 cm, vorzugsweise 1,5 bis 2,5 cm und in verschiedenen Längen-und Breitenabmessungen von z.B. 1 bis ca. 100 cm zur Anwendung kommen. Für einige Fälle wird auch die Abdeckung mit einer feuchtigkeitsdichten Folie zur Bildung einer feuchten Kammer von nöten sein, wobei die hautabgewandte Seite des Flächengebildes die wasserundurchlässige Schicht trägt.

Die erfindungsgemäßen nicht-immobilisierte Enzym-Trägermaterial-Kombinationen können in vielfacher Weise therapeutische Anwendung finden. Genannt seien insbesondere solche Erkrankungen, bei denen Hautpartien angegriffen bis zerstört sind.

Erwähnt seien beispielsweise

a) Brandwunden, z.B. Brandwunden 3. Grades

b) Durchliegegeschwüre (Dekubitus)

c) Geschwüre an den unteren Extremitäten (Ulcus cruris)

d) Gangrän

e) Verätzungen

f) Postoperative Wundtoilette bei Hauttransplantation

g) Traumatische Hautwunden

Beispiel 1:

8,3 g gereinigtes Trypsin, keimarm, werden gelöst in einer Mischung aus 9,4 g Glycerin, Ph. Eur., und 107,3 g gereinigtem Wasser, Ph. Eur., und anschließend steril filtriert durch ein Membranfilter mit 0,22/ µm Porenweite.

Unter aseptischen Bedingungen erfolgt dann eine gleichmäßige Befeuchtung von 10 Celluloseschwammtüchern, 2,5 mm dick, 83 × 100 mm, mit je 12,5 g der Enzymlösung.

Nach zweitägiger Trocknung im Laminar-Flow-Gerät bei Raumtemperatur werden die Präparate einseitig mit einer feuchtigkeitsundurchlässigen, handelsüblichen selbstklebenden Polyethylenfolie abgedeckt und hermetisch geschlossen verpackt. Die enzymatische Aktivität beträgt durchschnittlich 3 Protease-Einheiten nach FIP pro qcm.

Die erhaltene Wundabdeckung ist flexibel und kann in üblicher Weise auf den zu behandelnden Wunden fixiert werden. Nach 24 Stunden wird bei der Behandlung die Wundauflage erneuert.

Beispiel 2:

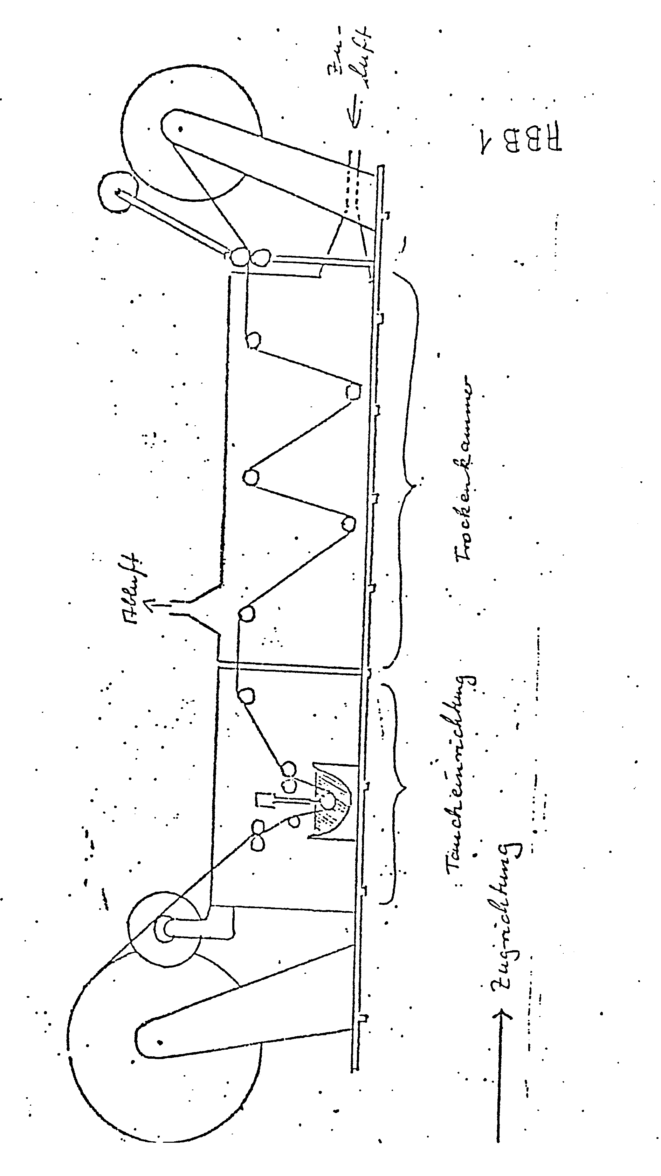

In einer kontinuierlich arbeitenden Tauchapparatur entsprechend ABB 1 werden 20 m lange und 10 cm breite Celluloseschwammvliesstücke (2,5 mm Dicke) durch Tauchen in eine steril filtrierte Enzymlösung folgende Zusammensetzung befeuchtet:

207,5 g gereinigtes Trypsin, keimarm, 235 g Glycerin, 6,25 g Eudragit ® E 30D und 1282,5 g Wasser, gereinigt, Ph.Eur.

Durch den Druck der dem Tauchschritt folgenden zylindrischen Walzen, wird die Flüssigkeitsaufnahme so eingestellt, daß im Endprodukt, nach Passage des Trockentunnels (80 - 90 Grad Celsius) 2,9 bis 3,2 Protease-Einheiten nach FIP/qcm enthalten sind.

Die feuchtigkeitsisolierende Abdeckung erfolgt durch Einziehen einer handelsüblichen selbsthaftenden Polyethylenfolie gemeinsam mit dem proteasehaltigen Schwammstreifen auf die Spule am Ende der Anlage.

Abschließend werden unter aseptischen Bedingungen 10 × 10 cm große Stücke abgeschnitten und hermetisch verpackt.

Beispiel 3:

10 Cellulose-Schwammstücke von 1 mm Schichtdicke (10 × 10 cm) werden unter einem Laminar-Flow-Gerät mit jeweils 15 g einer steril filtrierten Lösung getränkt, die folgendermaßen zusammengesetzt ist:

900 mg gereinigtes Trypsin, keimarm, 2 mg 5-Nitro-2 Furaldehydsemicarbazon (Nitrofurazon), 5 g Isopropanol, 1 g Glycerin, 3 mg Eudragit ® E30D und 9 g Wasser.

Die feuchten Schwammstücke werden getrocknet und entsprechend Beispiel 1 verpackt.

**Ansprüche**

1. Wundauflage auf Polysaccharidbasis zur topischen Behandlung die Haut betreffender und von der Haut her beeinflußbarer Erkrankungen,

dadurch gekennzeichnet,

daß sie aus einer schwammartigen Polysaccharidzubereitung, die therapeutisch wirksame Enzyme enthält, besteht.

2. Wundauflage nach Anspruch 1, dadurch gekennzeichnet, daß sie aus einer schwammartigen Cellulosezubereitung, die therapeutisch wirksame Enzyme enthält, besteht.

3. Wundauflage nach den Ansprüchen 1 und 2, dadurch gekennzeichnet, daß der schwammartige Enzymträger eine Saugfähigkeit von 1 500 bis 3 000 Gew.-% Wasser, bezogen auf die Trockenmasse der Cellulose besitzt.

4. Wundauflage nach den Ansprüchen 1 bis 3, dadurch gekennzeichnet, daß diese als Enzyme Proteasen enthält.

5. Wundauflage nach den Ansprüchen 1 bis 3, dadurch gekennzeichnet, daß diese als Enzyme Ribonucleasen oder/und Desoxyribonucleasen enthält.

6. Wundauflage nach den Ansprüchen 1 bis 5, dadurch gekennzeichnet, daß diese zusätzlich therapeutische Wirkstoffe enthält.

7. Wundauflage nach den Ansprüchen 1 bis 3, dadurch gekennzeichnet, daß sie mit dem Enzym Hyaluronidase, vorzugsweise in Kombination mit therapeutischen Wirkstoffen belegt ist.

Abluft

Zuluft

Taucheinrichtung    Trockenkammer

→ Zugrichtung

ABB 1